# EUROPÄISCHE PATENTANMELDUNG

(11) **EP 2 457 554 A1**
(43) Veröffentlichungstag der Anmeldung: **30.05.2012**
(21) Anmeldenummer: 10192468.6
(22) Anmeldetag: 24.11.2010
(51) Int. Cl.: A61K 8/34, A61Q 11/00, A23L 1/22, A23G 4/02

(54) **Menthol enthaltende Mischung**

(71) Anmelder: Symrise AG, 37603 Holzminden (DE)
(72) Erfinder: Sorge, Klaus, 37603, Holzminden (DE); Loges, Hubert, 37671, Höxter (DE); Machinek, Arnold, 37603, Holzminden (DE); Simchen, Ulrike, 37603, Holzminden (DE); Surburg, Horst, 37603, Holzminden (DE)
(74) Vertreter: Stilkenböhmer, Uwe Michael

(57) **Zusammenfassung**

Die vorliegende Erfindung betrifft d,I-Menthol sowie optional zusätzlich I-Menthol enthaltende Mischungen, die bei Normaldruck und einer Temperatur von 20 °C und höher flüssig sind. Des Weiteren betrifft die vorliegende Erfindung Produkte, die eine solche Mischung umfassen bzw. die durch Mischen oder in Kontakt bringen einer solchen Mischung mit weiteren Bestandteilen herstellbar sind, sowie neue Verfahren zur Herstellung mentholhaltiger Produkte.

## Beschreibung

Die vorliegende Erfindung betrifft primär eine neue Mischung, die bezogen auf das Gesamtgewicht der Mischung
- racemisches d,I-Menthol sowie optional zusätzlich I-Menthol, in einer Gesamtmenge von 72,0 bis 85,0 Gew.-%,
- d,I-Isomenthol in einer Menge von 7,5 bis 20,0 Gew.-%,
- ein erstes Alkylendiol sowie optional ein oder mehrere weitere Alkylendiole in einer Gesamtmenge von 7,0 bis 11,0 Gew.-%,
- weitere Inhaltsstoffe in einer Gesamtmenge von 0 bis 2,0 Gew.-%,
umfasst und bei einem Druck von 101325 Pa, nachfolgend auch "Normaldruck" genannt, und einer Temperatur von 20 °C und höher flüssig ist.

Die vorliegende Erfindung betrifft außerdem Produkte, die (i) eine erfindungsgemäße Mischung und weitere Bestandteile umfassen oder (ii) herstellbar sind durch Mischen oder in Kontakt bringen einer erfindungsgemäßen Mischung mit weiteren Bestandteilen, wobei die weiteren Bestandteile des Produkts kein Menthol und/oder Isomenthol sowie vorzugsweise kein Alkylendiol enthalten.

Die Erfindung betrifft des Weiteren ein Verfahren zur Herstellung eines mentholhaltigen Produkts, vorzugsweise eines erfindungsgemäßen Produkts.

Ein weiterer Aspekt der vorliegenden Erfindung betrifft die Verwendung von d,I-Isomenthol im Gemisch mit einem ersten Alkylendiol sowie optional einem oder mehreren weiteren Alkylendiolen als Mittel zur Erniedrigung des Schmelzpunktes von d,I-Menthol, optional in Gegenwart zusätzlichen I-Menthols, vorzugweise auf eine Temperatur von 20 °C oder niedriger bei einem Druck von 101325 Pa, d.h. bei Normaldruck.

Weitere Aspekte der vorliegenden Erfindung ergeben sich aus der folgenden Beschreibung, den Ausführungsbeispielen und den Ansprüchen.

Insbesondere wegen seiner herausragenden Eigenschaft, auf Haut und Schleimhäuten ein erfrischendes Kältegefühl zu erzeugen, gehört der Aromastoff Menthol zu den bedeutendsten Substanzen, die zur Aromatisierung von Produkten, insbesondere von Zahnpflegeprodukten, Kaugummis und Erfrischungsbonbons, eingesetzt werden.

Menthol wird vor allem in der enantiomeren-reinen I-Form (I-Menthol), je nach gewünschter Anwendung aber auch als Racemat (d,I-Form; d,I-Menthol) verwendet. (Reines) I-Menthol besitzt einen Schmelzpunkt von 42,5 °C. Der Schmelzpunkt von d,I-Menthol liegt im Bereich von 34 bis 36 °C (Beilstein, Hdb. Org. Chem. 4. Aufl., 2. Ergänzungswerk Bd. VI, Springer Berlin 1944, S. 40; S. 49).

Sowohl von I-Menthol, als auch von d,I-Menthol können durch schnelles Abkühlen zunächst metastabile Modifikationen mit niedrigeren Schmelzpunkten als den vorangehend genannten erzeugt werden. Die (metastabilen) Modifikationen gehen jedoch z. B. beim Lagern oder Tempern in stabilere Modifikationen mit höherem Schmelzpunkt über (F.E. Wright, J. Amer. Chem. Soc. 1917 (39), 1515; M. Kuhnert-Brandstätter, R. Ulmer, L. Langhammer, Arch. Pharm 1974 (307), 497 - 503).

Bei üblichen Lager-Temperaturen, d. h. insbesondere bei einer Temperatur im Bereich von 0 bis 30 °C, liegen I-Menthol und d,I-Menthol als kristalline Feststoffe vor.

Bei der Verarbeitung von Menthol, insbesondere von I-Menthol und d,I-Menthol, besteht je nach Verwendung häufig das Bedürfnis, Menthol nicht in fester, kristalliner Form, sondern in flüssiger Form einzusetzen. Denn der flüssige Aggregatzustand erlaubt vorteilhafterweise eine einfachere und genauere Dosierung bei der Zugabe von Menthol, insbesondere bei der Herstellung von Aromastoff-Zubereitungen (z.B. Aromakonzentraten), sowie eine bessere Einarbeitung in zu aromatisierende Medien bzw. Massen zur Herstellung aromatisierter Produkte.

Häufig wird Menthol zur Verwendung in flüssiger Form bisher zunächst geschmolzen und dann (in flüssiger Form) bei erhöhter Temperatur vorgehalten. Dieser Vorgang erfordert jedoch den Einsatz von speziellen Apparaturen und ist besonders energieaufwendig.

In der Aromastoff-verarbeitenden Industrie besteht daher das Bedürfnis, Menthol bzw. Menthol-Mischungen bereitzustellen, die bereits bei einer Umgebungstemperatur im Bereich von 20 °C in flüssigem Zustand verarbeitet werden können.

Hierfür sind im Stand der Technik bereits diverse Lösungsansätze beschrieben, welche jedoch zum Teil erhebliche Nachteile mit sich bringen.

Es ist beispielsweise grundsätzlich bekannt, Menthol in einem geeigneten Lösungsmittel aufzunehmen, um eine Lösung zu erhalten, die es ermöglicht, Menthol in flüssiger Form bei einer gewünschten Verarbeitstemperatur einzusetzen. Dieses Vorgehen birgt jedoch den Nachteil, dass hierfür relativ große Mengen an Lösungsmittel benötigt werden. Zum Einen wird durch die hierdurch bedingte Verdünnung von Menthol dessen gewünschte Wirkung herabgesetzt. Zum Anderen führt dies bei der Herstellung von Aromastoff-Zubereitungen (z.B. Aromakonzentraten) oder bei der Einarbeitung in zu aromatisierende Medien bzw. Massen zur Herstellung aromatisierter Produkte (z.B. Zahncremes und Kaugummis) zu diversen Problemen. So führt die Verwendung größerer Mengen an Lösungsmittel beispielsweise teilweise zu unerwünschten Veränderungen der Fließfähigkeit bzw. der Viskosität bestimmter Produkte.

So ergibt beispielsweise ein Zusatz von (lediglich) 10 Gew.-% des für Lebensmittelaromen zugelassenen Lösungsmittels 1,2-Propylenglykol zu d,I-Menthol eine Mischung, die bei Normaldruck (101325 Pa) und einer Temperatur von weniger als 25 °C als Feststoff vorliegt. Um eine Mischung zu erhalten, die bei Normaldruck bereits bei einer Temperatur von 20 °C flüssig vorliegt, ist es notwendig, die Menge an 1,2-Propylenglykol deutlich zu erhöhen. Dies birgt jedoch den Nachteil, dass das Lösungsmittel die sensorischen Eigenschaften von Menthol gegenüber dem Ausgangsmaterial, d. h. gegenüber reinem Menthol, in unerwünschtem Maße reduziert. Zudem kann ein derart hoher Lösungsmittel-Anteil zu Problemen bei der Formulierung von (Fertig-)Produkten führen.

Daher besteht in der Aromastoff-verarbeitenden Industrie insbesondere der Bedarf an Menthol enthaltenden Mischungen, die bei Normaldruck bereits bei einer Temperatur von 20 °C flüssig vorliegen und deren sensorische Wirkung gegenüber reinem Menthol, insbesondere gegenüber reinem I- oder d,I-Menthol, nicht oder zumindest nicht wesentlich herabgesetzt ist, wobei zudem der Lösungsmittelanteil, bezogen auf das Gesamtgewicht der Mischung, vorzugsweise im Bereich von 10 Gew.-% oder weniger liegt.

Eine im Stand der Technik bekannte Möglichkeit, den Schmelzpunkt von Menthol herabzusetzen, besteht in der Verwendung einer Mischung aus verschiedenen Menthol-Isomeren.

So kann man beispielsweise dem Schmelzdiagramm von d-Menthol und I-Menthol (s. M. Kuhnert-Brandstätter, R. Ulmer, L. Langhammer, Arch. Pharm 1974 (307), 497-503) entnehmen, dass der durch Mischen von d,I-Menthol und I-Menthol zu erhaltende niedrigste Schmelzpunkt (eutektischer Punkt) ca. 30 °C beträgt. Im Vergleich hierzu beträgt die Schmelztemperatur der reinen Enantiomeren 42,5 °C. Die am eutektischen Punkt vorliegende Zusammensetzung entspricht einem Mischungsverhältnis von 68 Gew-% I-Menthol und 32 Gew.-% d-Menthol, was wiederum einem Mischungsverhältnis von 64 Gew.-% (racemischem) d,I-Menthol und 36 Gew.-% (reinem) I-Menthol entspricht.

Aber auch durch Zugabe einer Lösungsmittelmenge von maximal 10 Gew-% 1,2-Propylenglykol zu der am eutektischen Punkt vorliegenden Zusammensetzung (siehe vorangehender Absatz) lässt sich der Schmelzpunkt nach eigenen Untersuchungen (siehe hierzu unten, Beispiel 1.3, dritte Mischung) lediglich auf 23 °C, nicht aber in den gewünschten Bereich von ≤ 20 °C absenken.

Ein weiterer Ansatz ist in JP 2008-220048 beschrieben. Darin wird berichtet, dass durch den Zusatz von d-Neomenthol zu I-Menthol ein Auskristallisieren von I-Menthol verhindert werden könne. Hierfür wird ein d-Neomenthol-Anteil von etwa 4 Gew.-%, bezogen auf den Gewichtsanteil von I-Menthol in der Zusammensetzung, empfohlen. Ein solches Vorgehen birgt jedoch den Nachteil, dass die kühlende Frischewirkung von Menthol durch die ausgeprägte erdig-muffige Note von Neomenthol spürbar beeinträchtigt wird (vgl. hierzu R. Emberger, R. Hopp, in Topics in Flavor Research, Hrsg. R.G. Berger, S. Nitz, P.Schreier, H. Eichhorn, Marzling-Hangenham 1985, S. 201-218). Ein Zusatz von d-Neomenthol zu I-Menthol bzw. d,I-Menthol ist daher aus sensorischen Erwägungen nicht wünschenswert.

Die primäre Aufgabe der vorliegenden Erfindung besteht daher im Ergebnis darin, eine Menthol enthaltende Mischung anzugeben, die unter Normaldruck bereits bei einer Temperatur von 20 °C in flüssigem Zustand vorliegt, wobei die weiteren Bestandteile dieser Mischung die sensorische Wirkung von Methanol vorzugsweise nicht oder allenfalls kaum beeinträchtigen. Weiter bevorzugt soll eine solche Mischung bereitgestellt werden, deren Lösungsmittel-Anteil nicht so hoch gewählt werden muss, dass die oben genannten Probleme auftreten.

Eine weitere Aufgabe der vorliegenden Erfindung besteht darin, neue mentholhaltige Produkte sowie ein Verfahren zur Herstellung eines neuen mentholhaltigen Produkts anzugeben.

Weitere Aufgaben im Zusammenhang mit der vorliegenden Erfindung ergeben sich aus der folgenden Beschreibung, den beigefügten Ausführungsbeispielen und den Patentansprüchen.

Die primäre Aufgabe der vorliegenden Erfindung wird gelöst durch eine Mischung, die bei einem Druck von 101325 Pa, d.h. bei Normaldruck, und einer Temperatur von 20 °C und höher flüssig ist, umfassend
- racemisches d,I-Menthol sowie optional zusätzlich I-Menthol, in einer Gesamtmenge von 72,0 bis 85,0 Gew.-%,
- d,I-Isomenthol in einer Menge von 7,5 bis 20,0 Gew.-%,
- ein erstes Alkylendiol sowie optional ein oder mehrere weitere Alkylendiole in einer Gesamtmenge von 7,0 bis 11,0 Gew.-%,
- weitere Inhaltsstoffe in einer Gesamtmenge von 0 bis 2,0 Gew.-%,
wobei die Gewichtsprozentangaben bezogen sind auf das Gesamtgewicht der Mischung.

Eine erfindungsgemäße Mischung umfasst demnach sowohl d-Menthol als auch I-Menthol, wobei die Menge an I-Menthol entweder eine gegenüber der Menge an d-Menthol äquimolare oder eine über-äquimolare Menge ist.

Eine erfindungsgemäße Mischung liegt (bei Normaldruck) bei einer Temperatur von 20 °C und höher, d. h. sowohl bei einer Temperatur von 20 °C, als auch bei einer höheren Temperatur, flüssig vor. Bei besonders bevorzugten Ausgestaltungen einer erfindungsgemäßen Mischung (wie im Rahmen des vorliegenden Textes beschrieben) werden teilweise sogar Schmelzpunkte von weniger als 20 °C erreicht. Dies war jedoch nicht zu erwarten, sondern vielmehr besonders überraschend, wie im Folgenden näher erläutert wird.

Isomenthol hat zwar ein für sensorische Zwecke geeigneteres Profil als d-Neomenthol (s. oben), besitzt jedoch Schmelzpunkte, die signifikant höher sind als die von Menthol. So liegt die Schmelztemperatur von d,I-Isomenthol (wie in einer erfindungsgemäßen Mischung enthalten) im Bereich von 52 bis 53 °C; die Schmelztemperatur von d-Isomenthol beträgt sogar 82,5 °C (Beilstein, Hdb. Org. Chem. 4. Aufl., 2. Ergänzungswerk Bd. VI, Springer Berlin 1944, S. 51). Daher war nicht zu erwarten, dass eine erfindungsgemäße Mischung (wie oben beschrieben) umfassend 7,5 bis 20 Gew.-% d,I-Isomenthol bei Normaldruck bereits bei einer Temperatur von 20 °C flüssig vorliegt. Insbesondere war nicht zu erwarten, dass der Zusatz von d,I-Isomenthol zur Erniedrigung des Schmelzpunktes der d,I-Menthol sowie optional zusätzlich I-Menthol enthaltenden Mischung in der erforderlichen Größenordnung beitragen kann. Denn wie wir in Vergleichsversuchen festgestellt haben, besitzt ein Gemisch aus 50 Gew.-% d,I-Menthol und 50 Gew.-% d,I-Isomenthol (ohne zusätzliches Lösungsmittel) eine Schmelztemperatur von 36,5 °C. Für ein Gemisch aus 80 Gew.-% d,I-Menthol und 20 Gew.-% d,I-Isomenthol (ohne zusätzliches Lösungsmittel) wurde unter Normaldruck eine Schmelztemperatur von 34,5 °C festgestellt. Auch die Schmelztemperaturen von Gemischen aus 25 bis 50 Gew.-% I-Menthol, 65 bis 40 Gew.-% d,I-Menthol und 10 Gew.-% d,I-Isomenthol lagen nach eigenen Untersuchungen im Bereich von 33 bis 34 °C.

Besonders überraschendend war, dass der Schmelzpunkt einer d,I-Menthol sowie optional zusätzlich I-Menthol enthaltenden Mischung durch Zugabe von 7,5 bis 20 Gew.-% d,I-Isomenthol und zudem 7,0 bis 11 Gew.-% Alkylendiol derart reduziert werden konnte, dass eine (erfindungsgemäße) Mischung erhalten wird, die bei Normaldruck (101325 Pa) bereits bei einer Temperatur von 20 °C flüssig vorliegt. Dies ist insofern überraschend, als dass beispielsweise ein Zusatz von 10 Gew.-% 1,2-Propylenglykol zu d,I-Menthol lediglich zu einer Schmelzpunkterniedrigung um maximal 10 °C und ein Zusatz von 10 Gew.-% 1,2-Propylenglykol zu einem eutektischen Gemisch aus d,I-Menthol und I-Menthol lediglich zu einer Schmelzpunkterniedrigung um maximal 7 °C führt (vgl. hierzu unten, Beispiele 1.2 und 1.3).

Bei den erfindungsgemäßen Mischungen (wie im vorliegenden Text beschrieben) wird der Schmelzpunkt gegenüber Mischungen aus I-Menthol (sowie gegebenenfalls d,I-Menthol) und Propylenglykol ohne zusätzliches d,I-Isomenthol vorteilhafterweise um etwa 15 °C oder mehr erniedrigt.

Für die Zwecke der vorliegenden Erfindung ist es besonders bevorzugt, dass die Menge an d,I-Isomenthol bezogen auf die Gesamtmenge an d,I-Menthol und gegebenenfalls I-Menthol 5 bis 25 Gew.-%, weiter bevorzugt 10 bis 20 Gew.-%, beträgt. In Kombination mit 7,5 bis 10 Gew.-% an Alkylendiol, z. B. 1,2-Propylenglykol, wird vorteilhafterweise eine erfindungsgemäße Mischung erhalten, deren Schmelzpunkt sogar unterhalb von 20 °C liegt.

Für die Zwecke der vorliegenden Erfindung besonders bevorzugt ist eine erfindungsgemäße Mischung, wobei das eine Alkylendiol bzw. eines, mehrere oder sämtliche der Alkylendiole ausgewählt sind aus der Gruppe bestehend aus Alkylenglykolen mit 2 bis 6 C-Atomen.

Besonders bevorzugt ist das bzw. eines, mehrere oder sämtliche der Alkylendiole ausgewählt aus der Gruppe bestehend aus Ethylenglykol, 1,2-Propylenglykol, 1,3-Propylenglykol, 1,2-Butylenglykol, 1,4-Butylenglykol und 1,2-Hexylenglykol.

Generell ist es bevorzugt, wenn das bzw. eines, mehrere oder sämtliche der Alkylendiole in der erfindungsgemäßen Mischung ausgewählt sind aus der Gruppe bestehend aus Ethylenglykol, Propylenglykol und Hexylenglykol.

Sofern eine erfindungsgemäße Mischung neben d,I-Menthol zusätzlich I-Menthol umfasst, ist es besonders bevorzugt, wenn das Gewichtsverhältnis von d,I-Menthol zu I-Menthol größer ist als 1 : 4, vorzugsweise größer als 1 : 2.

Gemäß einem besonders bevorzugten Aspekt der vorliegenden Erfindung liegt der Gewichtsanteil von I-Menthol, bezogen auf das Gesamtgewicht von d,I-Menthol und I-Menthol in der Mischung, in einer erfindungsgemäßen Mischung vorzugsweise im Bereich von 0 bis 80 Gew.-%, besonders bevorzugt im Bereich von 0 bis 60 Gew.-%. Weiter bevorzugt liegt der Anteil an I-Menthol in einer erfindungsgemäßen Mischung, bezogen auf das Gesamtgewicht der Mischung, im Bereich von 0 und 51 Gew.-%.

Gemäß einem weiteren Aspekt im Zusammenhang mit der vorliegenden Erfindung wird eine Mischung angegeben, bestehend aus oder umfassend eine Gesamtmenge von (racemischem) d,I-Menthol sowie optional zusätzlich (reinem) I-Menthol im Bereich von 61 bis 89 Gew.-%, vorzugsweise im Bereich von 71 bis 84 Gew.-%, eine Menge von d,I-Isomenthol im Bereich von 4 bis 24 Gew.-%, vorzugsweise im Bereich von 6 bis 24 Gew.-%, und eine Gesamtmenge von einem ersten Alkylendiol (z.B. 1,2-Propylenglycol) sowie optional einem oder mehreren weiteren Alkylendiolen im Bereich von 6 bis 19 Gew.-%, vorzugsweise eine Menge von 1,2-Propylenglycol im Bereich von 6 bis 11 Gew.-%. Für bevorzugte Ausgestaltungen einer solchen Mischung gilt das vorangehend Gesagte entsprechend.

Für die Zwecke der vorliegenden Erfindung besonders geeignet ist eine erfindungsgemäße Mischung (wie oben beschrieben) umfassend
- racemisches d,I-Menthol in einer Menge von 72,0 bis 85,0 Gew.-%,
- d,I-Isomenthol in einer Menge von 7,5 bis 20,0 Gew.-%,
- 1,2-Propylenglykol in einer Menge von 7,0 bis 11,0 Gew.-%,
- weitere Inhaltsstoffe in einer Gesamtmenge von 0 bis 2,0 Gew.-%,
wobei die Gewichtsprozentangaben bezogen sind auf das Gesamtgewicht der Mischung. Eine solche Mischung umfasst neben d,I-Menthol vorzugsweise kein zusätzliches (reines) I-Menthol.

Eine weitere bevorzugte Ausgestaltung der vorliegenden Erfindung betrifft eine erfindungsgemäße Mischung, umfassend
- racemisches d,I-Menthol in einer Menge von 48 bis 57 Gew.-%
- zusätzlich I-Menthol in einer Menge von 21,0 bis 32,0 Gew.-%,
- d,I-Isomenthol in einer Menge von 8,0 bis 14,0 Gew.-%,
- 1,2-Propylenglykol in einer Menge von 7,0 bis 11,0 Gew.-%,
- weitere Inhaltsstoffe in einer Gesamtmenge von 0 bis 2,0 Gew.-%,
wobei die Gewichtsprozentangaben bezogen sind auf das Gesamtgewicht der Mischung.

Gemäß einer bevorzugten Ausgestaltung der vorliegenden Erfindung umfasst eine erfindungsgemäße Mischung keine weiteren Inhaltsstoffe (0 Gew.-%), d.h. gemäß diesem Aspekt der vorliegenden Erfindung besteht eine erfindungsgemäße Mischung vorzugsweise aus racemischem d,I-Menthol sowie optional zusätzlich reinem I-Menthol in oben genannter Menge, d,I-Isomenthol in oben genannter Menge und einem oder mehreren Alkylendiolen in oben genannter Menge.

Die erfindungsgemäß vorliegenden Mischungen erlauben vorteilhafterweise eine leicht handhabbare und genaue Dosierung bei der Herstellung von Aromastoff-Zubereitungen, insbesondere Aromakonzentraten, und bieten zudem eine bessere Handhabung beim Einarbeiten in zu aromatisierende Medien und Massen. Ein weiterer Vorteil erfindungsgemäßer Mischungen besteht darin, dass diese lediglich 11 Gew.-% oder weniger an Alkylendiol umfassen, da Lösungsmittel in größeren Mengen, wie vorangehend beschrieben, in bestimmten Medien und Massen bzw. bei der Formulierung von (Fertig-)Produkten zu Problemen führen können.

Wie Vergleichstests zeigten, besteht ein weiterer Vorteil erfindungsgemäßer Mischungen darin, dass diese einen sensorischen Gesamteindruck vermitteln, welcher mit reinem d,I-Menthol vergleichbar ist.

Demnach eignen sich die erfindungsgemäßen Mischungen vorteilhafterweise besonders gut als Bestandteil von Aromastoff-Zubereitungen bzw. als Bestandteil zu aromatisierender Produkte. Die erfindungsgemäßen Mischungen eignen sich insbesondere zur Verwendung in Aromastoff-Zubereitungen, in kosmetischen Zubereitungen und der Ernährung und/oder dem Genuss dienenden Zubereitungen, insbesondere solchen, die herkömmlicherweise (kristallines) Menthol enthalten.

Ein weiterer Aspekt der vorliegenden Erfindung betrifft dementsprechend ein Produkt,
(i) umfassend eine Mischung nach einem der vorangehenden Ansprüche und weitere Bestandteile oder
(ii) herstellbar durch Mischen oder in Kontakt bringen einer Mischung nach einem der vorangehenden Ansprüche mit weiteren Bestandteilen,
wobei die weiteren Bestandteile des Produkts kein Menthol und/oder Isomenthol sowie vorzugsweise kein Alkylendiol enthalten.

Vorzugsweise ist das Produkt dabei ausgewählt aus der Gruppe bestehend aus Aromastoff-Zubereitungen, kosmetischen Zubereitungen, der Ernährung und/oder dem Genuss (z.B. Tabakprodukte) dienenden, vorzugsweise verzehr- bzw. gebrauchsfertigen Zubereitungen, insbesondere Getränken und kaubaren Nahrungsmitteln, insbesondere Kaugummis und Kaubonbons (z.B. Erfrischungsbonbons), Mundhygieneprodukten und Zahnpflegeprodukten, insbesondere Zahnpasta und zahnpflegende Kaugummis.

Ein erfindungsgemäßes Produkt ist demnach gemäß einem ersten Aspekt vorzugsweise eine Aromastoff-Zubereitung, welche neben der erfindungsgemäßen Mischung als einen oder mehrere weitere Bestandteile einen oder mehrere weitere Aromastoffe umfasst.

Eine erfindungsgemäße Aromastoff-Zubereitung ist vorzugsweise eine Aromastoff-Zubereitung, die zur Aromatisierung von Mundhygieneprodukten, Zahnpflegeprodukten und Kaugummis verwendet wird.

Gemäß einem weiteren Aspekt der vorliegenden Erfindung handelt es sich bei einem erfindungsgemäßen Produkt, wie oben beschrieben, vorzugsweise um eine kosmetische Zubereitung, eine der Ernährung und/oder dem Genuss dienende Zubereitung. Ein solches erfindungsgemäßes Produkt kann auch eine (erfindungsgemäße) Aromastoff-Zubereitung enthalten, die wiederum die erfindungsgemäße Mischung (wie oben beschrieben) umfasst. Bei einem solchen Produkt handelt es sich vorzugsweise um ein Mundhygieneprodukt, ein Zahnpflegeprodukt oder einen Kaugummi.

Besonders bevorzugt besitzt eine vorangehend beschriebene (erfindungsgemäße) Aromastoff-Zubereitung eine Eucalyptus-, eine Pfefferminz-, eine Spearmint, eine Zimt-, eine Wintergrün- und/oder eine Zitrus-Geschmacksnote.

Im Folgenden werden vorteilhafterweise mit einer erfindungsgemäßen Mischung zu kombinierende Aromastoffe (z. B. in einer erfindungsgemäßen Aromastoff-Zubereitung (wie oben beschrieben)) genannt:

Als zu verwendende Aromastoffe eignen sich grundsätzlich natürliche Rohstoffe wie aus Pflanzen gewonnene Extrakte und etherische Öle, bzw. daraus gewonnene Fraktionen und isolierte Stoffe, sowie auch einzelne synthetisch oder biotechnologisch gewonnene Aromastoffe.

Bevorzugte natürliche Rohstoffe sind gewählt aus der Gruppe bestehend aus: Pfefferminzöle, Krauseminzöle, Mentha-Arvensis-Öle, Anisöle, Nelkenöle, Citrusöle, Zimtrindenöle, Wintergrünöle, Cassiaöle, Davanaöle, Fichtennadelöle, Eucalyptusöle, Fenchelöle, Galbanumöle, Ingweröle, Kamillenöle, Kümmelöle, Rosenöle, Geraniumöle, Salbeiöle, Scharfgarbenöle, Sternanisöle, Thymianöle, Wacholderbeeröle, Rosmarinöle, Angelikawurzelöle, und die Fraktionen dieser Öle.

Bevorzugte Einzelverbindungen, die als weiterer Aromastoff bzw. weitere Aromastoffe mit einer erfindungsgemäßen Mischung eingesetzt werden können, sind vorzugsweise ausgewählt aus der Gruppe bestehend aus: Anethol, Menthon, Isomenthon, Menthylacetat, Menthofuran, Menthylmethylether, Mintlacton, Eucalyptol, Limonen, Eugenol, Pinen, Sabinenhydrat, 3-Octanol, Carvon, gamma-Octalacton, gamma-Nonalacton, Germacren-D, Viridiflorol, 1,3E,5Z-Undecatrien, Isopulegol, Piperiton, 2-Butanon, Ethylformiat, 3-Octylacetat, Isoamylisovalerianat, Hexanol, Hexanal, cis-3-Hexenol, Linalool, alpha-Terpineol, cis und trans Carvylacetat, p-Cymol, Thymol, 4,8-Dimethyl-3,7-nonadien-2-on, Damascenon, Damascone, Rosenoxid, Dimethylsulfid, Fenchol, Acetaldehyddiethylacetal, cis-4-Heptenal, Isobutyraldehyd, Isovaleraldehyd, cis-Jasmon, Anisaldehyd, Methylsalicylat, Myrtenylacetat, 8-Ocimenylacetat, 2-Phenylethylalkohol, 2-Phenylethylisobutyrat, 2-Phenylethylisovalerat, Zimtaldehyd, Geraniol, Nerol. Bei chiralen Verbindungen können die genannten Aromastoffe als Racemat, als einzelnes Enantiomer oder als enantiomerenangereicherte Mischungen vorliegen.

Bevorzugt einzusetzende zusätzliche Aromastoffe sind 1,8-Cineol (Eucaplyptol), Menthon, Carvon, Zimtaldehyd und/oder Methylsalicylat.

Es versteht sich, dass die genannten Aromastoffe in Form von Aromenmischungen eingesetzt werden können. Bevorzugt ist daher eine (erfindungsgemäße) Aromastoff-Zubereitung, umfassend als weitere(n) Bestandteil(e)
- Eucalyptusaromen, enthaltend Eucalyptol; und/oder
- Pfefferminzaromen, enthaltend Menthon; und/oder
- Spearmintaromen, enthaltend Carvon; und/oder
- Zimtaromen, enthaltend Zimtaldehyd; und/oder
- Wintergrünaromen, enthaltend Methylsalicylat.

In manchen Fällen sind erfindungsgemäße Produkte, insbesondere Aromastoff-Zubereitungen, bevorzugt, die zudem Aromastoffe enthalten, welche einen scharfen Geschmack oder eine Wärme- oder Hitzeempfindung auf Haut und Schleimhäuten oder ein Prickel-, bzw. Kribbelgefühl im Mund- und Rachenraum hervorrufen, wie z. B. Paprikapulver, Chili-Pfeffer-Pulver, Extrakte aus Paprika, Extrakte aus Pfeffer, Extrakte aus Chili-Pfeffer, Extrakte aus Ingwerwurzeln, Extrakte aus Paradieskörnern (Aframomum melegueta), Extrakte aus Parakresse (Jambu-Oleoresin; Spilanthes acmella, bzw. Spilanthes oleracea), Extrakte aus Japanischem Pfeffer (Zanthoxylum piperitum), Extrakte aus Kaempferia galanga, Extrakte aus Alpinia galanga, Extrakte aus Wasserpfeffer (Polygonium hydropiper), Capsaicin, Dihydrocapsaicin, Gingerol, Paradol, Shogaol, Piperin, Saanshool-I, Saanshool-II, Sanshoamid, Spilanthol, Carbonsäure-N-Vanillylamide, insbesonders Nonansäure-N-vanillylamid, 2-Nonensäureamide, insbesonders 2-Nonensäure-N-isobutylamid, 2-Nonensäure-N-4-hydroxy-3-methoxyphenylamid, Alkylether von 4-Hydroxy-3-methoxybenzylalkohol, insbesonders 4-Hydroxy-3-methoxybenzyl-n-butylether, Alkylether von 3-Hydroxy-4-methoxybenzylalkohol, Alkylether von 3,4-Dimethoxybenzylalkohol, Alkylether von 3-Ethoxy-4-hydroxybenzylalkohol, Alkylether von 3,4-Methylendioxybenzylalkohol, Acetale von Vanillin, Acetale von Ethylvanillin, Acetale von Isovanillin, (4-Hydroxy-3-methoxyphenyl)essigsäureamide, insbesonders (4-Hydroxy-3-methoxyphenyl)essigsäure-N-n-octylamid, Allylisothiocyanat, Nicotinaldehyd, Methylnicotinat, Propylnicotinat, 2-Butoxyethylnicotinat, Benzylnicotinat, 1-Acetoxychavicol.

Darüber hinaus lassen sich erfindungsgemäße Mischungen (wie oben beschrieben) hervorragend mit Stoffen kombinieren, die eine physiologische Kühlwirkung auf Haut und/oder Schleimhäuten erzeugen.

Dementsprechend enthält ein erfindungsgemäßes Produkt vorzugsweise zusätzlich einen oder mehrere (weitere) physiologische Kühlwirkstoffe. Der bzw. diese weiteren physiologischen Kühlwirkstoffe werden dabei vorzugsweise ausgewählt aus der Gruppe bestehend aus: Menthylether (z. B. (I-Menthoxy)-1,2-propandiol, (I-Menthoxy)-2-methyl-1,2-propandiol, I-Menthyl-methylether), Menthylester (z. B. Menthylformiat, Menthylacetat, Menthylisobutyrat, Menthyllactate, vorzugsweise L-Menthyllactat, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z. B. Menthylalkylcarbonate, Menthylpropylenglycolcarbonat, Menthylethylenglycolcarbonat, Menthylglycerincarbonat oder deren Mischungen), die Halbester von Mentholen mit einer Dicarbonsäure oder deren Derivate (z. B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), Menthancarbonsäureamide (z. B. Menthancarbonsäure-N-ethylamid [WS-3], Menthancarbonsäure-N-(4-methoxyphenyl)-amid [WS-12], N^{α}-(Menthancarbonyl)glycinethylester [WS-5], Menthancarbonsäure-N-(4-cyanophenyl)amid, Menthancarbonsäure-N-(alkoxyalkyl)amide), Menthon und Menthonderivate (z. B. Menthonketale wie L-Menthonglycerinketal), 2,3-Dimethyl-2-(2-propyl)-butansäurederivate (z. B. 2,3-Dimethyl-2-(2-propyl)-butansäure-N-methylamid [WS-23]), Isopulegol oder seine Ester (I-(-)-Isopulegol, I-(-)-Isopulegolacetat), Menthanderivate (z. B. p-Menthan-3,8-diol), Cubebol oder synthetische oder natürliche Mischungen, enthaltend Cubebol, Pyrrolidonderivate von Cycloalkyldionderivaten (z. B. 3-Methyl-2(1-pyrrolidinyl)-2-cyclopenten-1-on) oder Tetrahydropyrimidin-2-one (z. B. Icilin oder verwandte Verbindungen, wie in WO 2004/026840 beschrieben), N-(4-cyanomethylphenyl)-p-menthancarboxamid, N-(2-(Pyridin-2-yl)ethyl)-3-p-menthancarboxamid, Essigsäure-2-(methylamino)-2-oxo-, 5-methyl-2-(1-methylethyl)cyclohexylester, Essigsäure-2-(ethylamino)-2-oxo-, 5-methyl-2-(1-methylethyl)cyclohexylester. Kühlwirkstoffe auf Basis eines Menthol-Grundgerüstes sind dabei bevorzugt, insbesondere Derivate des I-Menthols.

Bevorzugt ist der Einsatz eines oder mehrerer weiterer physiologischer Kühlwirkstoffe, die zwar eine physiologische Kühlwirkung verursachen, jedoch gleichzeitig keine bzw. keine relevante geschmackliche Wirkung verursachen. Bevorzugte physiologische Kühlwirkstoffe sind deshalb ausgewählt aus der Gruppe bestehend aus: Menthylether (z. B. (I-Menthoxy)-1,2-propandiol, (I-Menthoxy)-2-methyl-1,2-propandiol), polarere Menthylester (z. B. Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z. B. Menthylpropylenglycolcarbonat, Menthylethylenglycolcarbonat, Menthylglycerincarbonat), die Halbester von Mentholen mit einer Dicarbonsäure oder deren Derivate (z. B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), nicht erfindungsgemäße Menthancarbonsäureamide (z. B. Menthancarbonsäure-N-ethylamid [WS-3], Menthancarbonsäure-N-(4-methoxyphenyl)-amid [WS-12], N^{α}-(Menthancarbonyl)glycinethylester [WS-5], Menthancarbonsäure-N-(4-cyanophenyl)amid, Menthancarbonsäure-N-(alkoxyalkyl)amide), Menthonderivate (z. B. L-Menthonglycerinketal), 2,3-Dimethyl-2-(2-propyl)-butansäurederivate (z. B. 2,3-Dimethyl-2-(2-propyl)-butansäure-N-methylamid), Pyrrolidonderivate von Cycloalkyldionderivaten (z. B. 3-Methyl-2(1-pyrrolidinyl)-2-cyclopenten-1-on), 2,2,2-Trialkylessigsäureamide (z. B. 2,2-Diisopropylpropionsäuremethylamide) oder Tetrahydropyrimidin-2-one (z. B. Icilin, Icilin-Derivate oder verwandte Verbindungen, die in WO 2004/026840 beschrieben sind).

Substanzen und Hilfsstoffe, die ein erfindungsgemäße Produkt (zusätzlich) enthalten kann, sind beispielsweise:

Konservierungsmittel, vorzugsweise die in US 2006/0089413 genannten, Abrasiva, Antiakne-Mittel und Mittel zu Sebumreduktion, vorzugsweise die in WO 2008/046791 genannten, Mittel gegen Hautalterung, vorzugsweise die in WO 2005/123101 genannten, antibakterielle Mittel, Anticellulitis-Mittel, Antischuppen-Mittel, vorzugsweise die in WO 2008/046795 genannten, entzündungshemmende Mittel, irritationsverhindernde Mittel, Antiirritantien (antiinflammatorische, irritationshemmende und irritationsverhindernde Mittel), vorzugsweise die in WO 2007/042472 und US 2006/0089413 genannten, antimikrobielle Mittel, vorzugsweise die in WO 2005/123101 genannten, Antioxidantien, vorzugsweise die in WO 2005/123101 genannten, Adstringentien, antiseptische Mittel, Antistatika, Binder, Puffer, Trägermaterialien, vorzugsweise die in WO 2005/123101 genannten, Chelatbildner, vorzugsweise die in WO 2005/123101 genannten, Zellstimulantien, reinigende Mittel, pflegende Mittel, Enthaarungsmittel, oberflächenaktive Substanzen, deodorierende Mittel und Antiperspirantien, vorzugsweise die in WO 2005/123101 genannten, Weichmacher, Emulgatoren, vorzugsweise die in WO 2005/123101 genannten, Enzyme, ätherische Öle, vorzugsweise die in US 2008/0070825 genannten, Insektenrepellentien, vorzugsweise die in WO 2005/123101 genannten, Fasern, Filmbildner, Fixateure, Schaumbildner, Schaumstabilisatoren, Substanzen zum Verhindern des Schäumens, Schaumbooster, Fungizide, gelierende Mittel und gelbildende Mittel, vorzugsweise die in WO 2005/123101 genannten, Haarpflegemittel, Haarverformungsmittel, Haarglättungsmittel, Feuchtigkeitsregulatoren (feuchtigkeitsspendende, anfeuchtende und/oder feuchthaltende Substanzen), vorzugsweise die in WO 2005/123101 genannten, Osmolyte, vorzugsweise die in WO 2005/123101 genannten, kompatible Solute, vorzugsweise die in WO 01/76572 und WO 02/15686 genannten, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, vorzugsweise die in WO 2008/046676 genannten, Pulver, Proteine und Proteinhydrolysate, vorzugsweise die in WO 2005/123101 und WO 2008/046676 genannten, rückfettende Mittel, abschleifende Mittel, hautberuhigende Mittel, hautreinigende Mittel, hautpflegende Mittel, hautheilende Mittel (skin repair agents), vorzugsweise enthaltend Cholesterin und/oder Fettsäuren und/oder Ceramide und/oder Pseudoceramide, dabei bevorzugt die in WO 2006/053912 genannten, Hautaufhellungsmittel, vorzugsweise die in WO 2007/110415 genannten, hautschützende Mittel, hauterweichende Mittel, hautkühlende Mittel, vorzugsweise die in WO 2005/123101 genannten, hautwärmende Mittel, vorzugsweise die in WO 2005/123101 genannten, Stabilisatoren, UV-absorbierende Mittel und UV-Filter, vorzugsweise die in WO 2005/123101 genannten, Benzyliden-betadicarbonylverbindungen, vorzugsweise die in WO 2005/107692 genannten, alpha-Benzoylzimtsäurenitrile, vorzugsweise die in WO 2006/015954 genannten, AhR-Rezeptor-Antagonisten, vorzugsweise die in WO 2007/128723 und WO 2007/060256 genannten, Waschmittel, Weichspüler, suspendierende Mittel, Hautbräunungsmittel, vorzugsweise die in WO 2006/045760 genannten, Verdickungsmittel, Vitamine, vorzugsweise die in WO 2005/123101 genannten, Öle, Wachse und Fette, vorzugsweise die in WO 2005/123101 genannten, Phospholipide, vorzugsweise die in WO 2005/123101 genannten, Fettsäuren (gesättigte Fettsäuren, ein- oder mehrfach ungesättigte Fettsäuren, α-Hydroxysäuren, Polyhydroxyfettsäuren), vorzugsweise die in WO 2005/123101 genannten, Verflüssiger, Farbstoffe und farbschützende Mittel sowie Pigmente, vorzugsweise die in WO 2005/123101 genannten, Antikorrosiva, Aromen und Geschmackstoffe sowie Riechstoffe, vorzugsweise die in S. Arctander, Perfume and Flavor Chemicals, Eigenverlag, Montclair, N.J., 1969 und Surburg, Panten, Common Fragrance and Flavor Materials, 5th Edition, Wiley-VCH, Weinheim 2006 aufgeführten, insbesondere die in US 2008/0070825 explizit genannten, Alkohole und Polyole, vorzugsweise die in WO 2005/123101 genannten, Tenside, vorzugsweise die in WO 2005/123101 genannten, tierische Extrakte, Hefeextrakte, Extrakte von Algen oder Mikroalgen, Elektrolyte, Verflüssiger, organische Lösungsmittel, vorzugsweise die in WO 2005/123101 genannten, oder Silikone und Silikonderivate, vorzugsweise die in WO 2008/046676 genannten.

Ferner vorteilhaft sind erfindungsgemäße Produkte, die oral einnehmbar sind, z. B. in Form von Tabletten (z. B. Filmtabletten), Dragees, Kapseln (z. B. Gelatinekapseln), Granulaten, Säften, Lösungen, Emulsionen, Mikroemulsionen, Sprays oder in sonstiger Form oral konsumierbarer Produkte oder in Form von Lebensmitteln, welche zusammen mit weiteren darin enthaltenen entsprechenden Wirkstoffen "Schönheit von Innen" ("beauty from inside") bewirken.

Weiterhin können die erfindungsgemäßen Mischungen oder die oben beschriebenen Produkte, insbesondere Aromastoff-Zubereitungen, in verkapselter Form vorliegen, wobei diese vorzugsweise verkapselt sind mit einem festen Hüllmaterial, das vorzugsweise ausgewählt ist aus Stärken, abgebaute oder chemisch oder physikalisch modifizierte Stärken (insbesondere Dextrine und Maltodextrine), Gelatinen, Gummi Arabicum, Agar-Agar, Ghatti-Gummi, Gellan Gum, modifizierte und nicht modifizierte Cellulosen, Pullulan, Curdlan, Carrageenane, Alginsäure, Alginate, Pektin, Inulin, Xanthan Gum und Mischungen zweier oder mehrerer der genannten Substanzen.

Vorzugsweise wird das feste Hüllmaterial gewählt aus Gelatine (vorteilhaft sind Schweine-, Rinder-, Hühner- und/oder Fischgelatinen und Mischungen davon, vorzugsweise umfassend mindestens eine Gelatine mit einem Bloom-Wert von größer oder gleich 200, bevorzugt mit einem Bloom-Wert von größer oder gleich 240), Maltodextrin (vorzugsweise aus Mais, Weizen, Tapioka oder Kartoffel gewonnen, bevorzugte Maltodextrine weisen einen DE-Wert von 10 bis 20 auf), modifizierte Cellulose (z. B. Celluloseether), Alginate (z. B. Na-Alginat), Carrageenan (beta-, iota-, lambda- und/oder kappa- Carrageenan), Gummi Arabicum, Curdlan und/oder Agar-Agar. Gelatine wird insbesondere wegen ihrer guten Verfügbarkeit in unterschiedlichen Bloom-Werten bevorzugt verwendet. Besonders bevorzugt namentlich zur oralen Verwendung sind nahtlose Gelatine- oder Alginatkapseln, deren Hülle sich im Mund sehr schnell auflöst bzw. beim Kauen zerplatzt. Die Herstellung kann beispielsweise erfolgen wie beschrieben in EP 0 389 700, US 4,251,195, US 6,214,376, WO 03/055587 oder WO 2004/050069.

Wie vorangehend erwähnt, handelt es sich gemäß einem Aspekt der vorliegenden Erfindung bei einem erfindungsgemäßen Produkt vorzugsweise um eine kosmetische Zubereitung. Eine erfindungsgemäß bevorzugte kosmetische Zubereitung stellt eine dermatologische Zubereitung dar, die (abgesehen von der enthaltenen, erfindungsgemäßen Mischung) wie üblich zusammengesetzt ist und vorzugsweise dem kosmetischen, insbesondere dermatologischen Lichtschutz, zur Behandlung, der Pflege und der Reinigung der Haut und/oder der Haare oder als Schminkprodukt in der dekorativen Kosmetik dient. Entsprechend können derartige Zubereitungen, je nach ihrem Aufbau beispielsweise als Hautschutzcreme, Tages- oder Nachtcreme, Augencreme, Sonnenschutz- oder After-sun-Lotion, Nährcreme, Pflegemaske, Gel-Pads, Gesichtswasser, feuchte Pflege- und Reinigungstücher, Reinigungsmilch, Reinigungsseife, Schaum- oder Duschbad, Deodorant, Antitranspirant, Haarshampoo, Haarpflegemittel, Haarconditioner, Haarcoloration, Haarstylingmittel verwendet werden und dabei bevorzugt als Emulsion, Lotion, Milch, Fluid, Creme, Hydrodispersionsgel, Balm, Spray, alkoholische oder wässrig/alkoholische Lösung, Schaum, Puder, Flüssigseife, Seifenstück, Shampoo, Roll-on, Stick oder Make-up vorliegen. In Haarbehandlungsmitteln richtet sich die Verwendung vorzugsweise auf den Haarboden oder die Kopfhaut.

Zur Anwendung werden die erfindungsgemäßen kosmetischen Zubereitungen in der für Kosmetika üblichen Weise auf die Haut und/oder die Haare in ausreichender Menge aufgebracht.

Entsprechend können erfindungsgemäße kosmetische Zubereitungen, je nach ihrem Aufbau, vorzugsweise verwendet werden als Hautpflegemittel, z. B. Hautschutzcreme, Tages- oder Nachtcreme, Sonnenschutzmittel, der After-sun-Präparat (z. B. After-sun-Lotion) Pflegemaske, Gel-Pads, Gesichtswasser, feuchte Pflege- und/oder Tränkungslösung, z. B. für kosmetische Tücher, Reinigungstücher, Reinigungsseife, Schaum- oder Duschbad, Flüssigseife, Seifenstück, Shampoo (z. B. 2-in-1 Shampoo, Anti-Schuppen-Shampoo, Babyshampoo, Shampoo für trockene Kopfhaut, Shampookonzentrat), Haarpflegemittel, Haarconditioner, Haarcoloration, Haarspülung, Haarstylingmittel (z. B. Haargel), Deodorant, Antitranspirans (z. B. Roll-on oder Stick), Rasierpräparat (z. B. Aftershave Balm, Preshave- oder Aftershave-Lotion) oder als Make-Up Entferner.

Dabei liegt eine erfindungsgemäße kosmetische Zubereitung in Form einer Emulsion, Lotion, Fluid, Creme, Mikroemulsion, Gel (z. B. Hydro- oder Hydrodispersionsgel), Balsam, Pumpspray, alkoholische oder wässrig/alkoholische Lösung vor.

Ein weiterer Aspekt der vorliegenden Erfindung betrifft ein Verfahren zur Herstellung eines mentholhaltigen Produkts, vorzugweise eines erfindungsgemäßen Produkts (wie oben beschrieben), umfassend die folgenden Schritte:
- Bereitstellen oder Herstellen einer erfindungsgemäßen Mischung (wie oben beschrieben),
- Mischen oder in Kontakt bringen der Mischung mit weiteren Zutaten,
wobei die weiteren Zutaten kein Menthol und/oder Isomenthol sowie vorzugsweise kein Alkylendiol enthalten.

Für bevorzugte Ausgestaltungen einer (erfindungsgemäßen) Mischung gilt das vorangehend Gesagte entsprechend. Zudem gilt für die weiteren Zutaten das vorangehend im Zusammenhang mit in erfindungsgemäßen Produkten enthaltenen weiteren Bestandteilen Gesagte entsprechend.

Erfindungsgemäß bevorzugt ist ein wie oben beschriebenes Verfahren, wobei das Mischen oder in Kontakt bringen der Mischung mit den weiteren Zutaten bei einer Temperatur erfolgt, die höher ist als der Schmelzpunkt der Mischung, aber niedriger ist als 25 °C, vorzugsweise niedriger ist als 23 °C.

Weiter bevorzugt ist ein Verfahren zur Herstellung einer Mischung, die bei einem Druck von 101325 Pa, d.h. bei Normaldruck, und einer Temperatur von 20 °C und höher flüssig ist und racemisches d,I-Menthol sowie optional zusätzliches I-Menthol umfasst, in einer Gesamtmenge von 72,0 bis 85,0 Gew.-% bezogen auf das Gesamtgewicht der Mischung, vorzugweise zur Herstellung einer erfindungsgemäßen Mischung (wie oben beschrieben),
mit folgenden Schritten:
- Bereitstellen oder Herstellen von insgesamt 72,0 bis 85,0 Gewichtsteilen des racemischen d,I-Menthols sowie optional des zusätzlichen I-Menthols,
- Mischen des racemischen d,I-Menthols sowie gegebenenfalls des zusätzlichen I-Menthols mit:
- d,I-Isomenthol in einer Menge von 7,5 bis 20,0 Gewichtsteilen,
- einem ersten Alkylendiol sowie optional einem oder mehreren weiteren Alkylendiolen in einer Gesamtmenge von 7,0 bis 11,0 Gewichtsteilen,
- weiteren Inhaltsstoffen in einer Gesamtmenge von 0 bis 2,0 Gewichtsteilen,
wobei die Mengenverhältnisse so eingestellt werden, dass die resultierende Mischung bei einem Druck von 101325 Pa und einer Temperatur von 20 °C und höher flüssig ist.

Besonders bevorzugt gilt dabei das für das bzw. die eingesetzten Alkylendiole sowie das für die bevorzugt einzusetzenden Mengen oben Gesagte entsprechend.

Dementsprechend ist ein erfindungsgemäßes Verfahren (wie oben beschrieben) besonders bevorzugt, wobei das erste Alkylendiol bzw. eines, mehrere oder sämtliche der Alkylendiole ausgewählt sind aus der Gruppe bestehend aus Alkylenglykolen mit 2 bis 6 C-Atomen.

Besonders bevorzugt ist das bzw. eines, mehrere oder sämtliche der Alkylendiole ausgewählt aus der Gruppe bestehend aus Ethylenglykol, 1,2-Propylenglykol, 1,3-Propylenglykol, 1,2-Butylenglykol, 1,4-Butylenglykol und 1,2-Hexylenglykol.

Generell bevorzugt ist ein erfindungsgemäßes Verfahren (wie oben beschrieben), wobei das, eines, mehrere oder sämtliche der Alkylendiole ausgewählt sind aus der Gruppe bestehend aus Ethylenglykol, Propylenglykol und Hexylenglykol.

Besonders bevorzugt ist ein wie oben beschriebenes, erfindungsgemäßes Verfahren, wobei das Gewichtsverhältnis von d,I-Menthol zu I-Menthol größer ist als 1 : 4, vorzugsweise größer als 1 : 2. Im Übrigen gilt das für das Verhältnis von d,I-Menthol zu I-Menthol oben Gesagte entsprechend.

Ein weiterer Aspekt der vorliegenden Erfindung betrifft die Verwendung von d,I-Isomenthol im Gemisch mit einem ersten Alkylendiol sowie optional einem oder mehreren weiteren Alkylendiolen als Mittel zur Erniedrigung des Schmelzpunktes von d,I-Menthol, optional in Gegenwart zusätzlichen I-Menthols, vorzugweise auf eine Temperatur von 20 °C oder niedriger bei einem Druck von 101325 Pa.

Nachfolgend wir die Erfindung anhand von Beispielen näher erläutert. Sofern nicht anders angegeben, beziehen sich dabei alle Mengenangaben auf das Gewicht.

### Beispiele:

### 1. Herstellung von erfindungsgemäßen Mischungen und Vergleichsmischungen und Bestimmung der Schmelzpunkte

### 1.1 Allgemeine Vorgehensweise:

### Herstellung der erfindungsgemäßen Mischungen bzw. der Vergleichsmischungen:

Die gewünschten Mengen an d,I-Menthol, I-Menthol und d,I-Isomenthol (s. unten, 1.2 - 1.6) werden aufgeschmolzen, zusammengegeben und (noch im flüssigen Zustand) mit 1,2-Propylenglycol versetzt.

Sämtliche der untenstehenden Mengenangaben sind als Gewichtsprozent, bezogen auf das Gesamtgewicht der jeweiligen Mischung, zu verstehen.

### Bereitstellen der Proben zur Bestimmung der Schmelzpunkte:

Nach Abkühlen der Mischung auf Raumtemperatur wird mit einer geringen Menge an d,I-Menthol-Kristallen angeimpft und gegebenenfalls die Mischung abgekühlt, bis eine merkliche Kristallisation einsetzt. Anschließend wird die Mischung für 24h bei 5 °C im Kühlschrank gelagert.

Von den Mischungen wird als Probe jeweils ca. 1g in ein rundes 10 g-Probengefäß mit 1,5 cm Durchmesser abgefüllt. Die Probengefäße werden dann so in ein auf 15 °C temperiertes Wasserbad gestellt, dass die Probenmenge sich unterhalb der Wasseroberfläche befindet.

### Bestimmung der Schmelzpunkte:

Über einen Zeitraum von 10 min wird die Temperatur des Wasserbades in einem ersten Schritt um 0,5 °C erhöht und anschließend für 30 min bei dieser Temperatur gehalten. Anschließend wird die Temperatur des Wasserbads in weiteren Schritten in entsprechenden Intervallen um jeweils 0,5 °C weiter erhöht. Als Schmelzpunkt der Mischung bzw. der Probe wurde die Temperatur definiert, bei der keine Kristallreste mehr in der Probe zu sehen waren.

Folgende Mischungen wurden (wie vorangehend beschrieben) hergestellt, wobei die genannten Schmelzpunkte (wie vorangehend beschrieben) gemessen wurden:

### 1.2 Mischungen aus d,I-Menthol und 1,2-Propylenglycol (Vergleichsmischungen)

| **d,I-Menthol** | **1,2-Propylenglycol** | **Schmp. [°C]** |
|---|---|---|
| 95 | 5 | 28 |
| 92,5 | 7,5 | 27,5 |
| 90 | 10 | 25 |

### 1.3 Mischungen aus d,I-Menthol, I-Menthol und 1,2-Propylenglycol (Vergleichsmischungen)

| **d,I-Menthol** | **I-Menthol** | **1,2-Propylenglycol** | **Schmp. [°C]** |
|---|---|---|---|
| 60,8 | 34,2 | 5 | 27,5 |
| 59,2 | 33,3 | 7,5 | 24,5 |
| 57,6 | 32,4 | 10 | 23 |

### 1.4 Mischungen aus d,I-Menthol, I-Menthol und d,I-Isomenthol (Vergleichsmischungen)

| **I-Menthol** | **d,I-Menthol** | **d,I-Isomenthol** | **Schmp. [°C]** |
|---|---|---|---|
| 0 | 50 | 50 | 36,5 |
| 0 | 80 | 20 | 34,5 |
| 25 | 60 | 15 | 33 |
| 35 | 55 | 10 | 33,5 |
| 50 | 42 | 8 | 34 |

### 1.5 Mischungen aus d,I-Menthol, d,I-Isomenthol und I,2-Propylenglycol (erfindungsgemäße Mischungen Nr. 2, 3 und 5, Vergleichsmischungen Nr. 1, 4 und 6-9)

| **Mischung Nr.** | **d,I-Menthol** | **d,I-Isomenthol** | **1,2-Propylenglycol** | **Schmp. [°C]** |
|---|---|---|---|---|
| 1 | 85,5 | 4,5 | 10 | 22,5 |
| 2 | 81 | 9 | 10 | 18,5 |
| 3 | 83,25 | 9,25 | 7,5 | 19,5 |
| 4 | 85,5 | 9,5 | 5 | 23 |
| 5 | 72 | 18 | 10 | 19,5 |
| 6 | 76 | 19 | 5 | 26 |
| 7 | 67,5 | 22,5 | 10 | 22 |
| 8 | 63 | 27 | 10 | 22,5 |
| 9 | 58,5 | 31,5 | 10 | 23 |

### 1.6 Mischungen aus I-Menthol, d,I-Menthol, d,I-Isomenthol und 1,2-Propylenglycol

| **I-Menthol** | **d,I-Menthol** | **d,I-Isomenthol** | **1,2-Propylenglycol** | **Schmp. [°C]** |
|---|---|---|---|---|
| 23 | 55 | 12 | 10 | 20 |
| 30 | 50 | 10 | 10 | 20 |

### 2. Sensorische Bewertung erfindungsgemäßer Mischungen

### 2.1 Direkter Vergleich einer erfindungsgemäßer Mischung gegen d,I-Menthol (Vergleichsprobe)

In eine Zuckermasse (Fondant) wurden
(1.) d,I-Menthol (Vergleichsprobe) bzw.
(2.) eine erfindungsgemäße Mischung, bestehend aus 81 Gew-% d,I-Menthol, 9 Gew-% d,I-Isomenthol und 10 Gew.-% 1,2-Propylenglycol (Beispiel 1.5, Nr. 2), in einer Konzentration von 0,05 % eingearbeitet.

Die Verkostung der Zuckermassen durch ein trainiertes Panel ergab für beide Proben eine sehr ähnliche Bewertung: typisch, mentholig, frisch. Bei der Zuckerprobe enthaltend die erfindungsgemäße Mischung konnten zudem vorteilhafterweise keine störenden Noten oder eine verminderte Frischwirkung festgestellt werden.

### 2.2 Vergleich einer erfindungsgemäßer Mischung gegen d,I-Menthol (Vergleichsprobe) in einem aromatisierten Fertigprodukt

In eine Standard-Silica-Zahnpastamasse mit einem Saccharingehalt von 0,2 Gew.-% wurden jeweils 0,5 Gew.-% eines Standard-Zahnpasta-Aromas (Optamint^{®} FL010529AA, Symise AG, Holzminden) und
(1.) 0,5 Gew.-% d,I-Menthol bzw.
(2.) 0,5 Gew.-% einer erfindungsgemäßen Mischung, bestehend aus 81 Gew-% d,I-Menthol, 9 Gew-% d,I-Isomenthol und 10 Gew.-% 1,2-Propylenglycol (Beispiel 1.5, Nr. 2),

eingearbeitet. Die Zahnpasten wurden unter Praxis-Bedingungen durch ein geschultes Panel getestet. Die Bewertung ergab für beide Proben: vergleichbar, sauber, mentholig, stark und frisch.

## Patentansprüche

1. Mischung, die bei einem Druck von 101325 Pa und einer Temperatur von 20 °C und höher flüssig ist, umfassend
- racemisches d,I-Menthol sowie optional zusätzlich I-Menthol, in einer Gesamtmenge von 72,0 bis 85,0 Gew.-%,
- d,I-Isomenthol in einer Menge von 7,5 bis 20,0 Gew.-%,
- ein erstes Alkylendiol sowie optional ein oder mehrere weitere Alkylendiole in einer Gesamtmenge von 7,0 bis 11,0 Gew.-%,
- weitere Inhaltsstoffe in einer Gesamtmenge von 0 bis 2,0 Gew.-%,
wobei die Gewichtsprozentangaben bezogen sind auf das Gesamtgewicht der Mischung.

2. Mischung nach Anspruch 1, wobei das eine Alkylendiol bzw. eines, mehrere oder sämtliche der Alkylendiole ausgewählt sind aus der Gruppe bestehend aus Alkylenglykolen mit 2 bis 6 C-Atomen.

3. Mischung nach einem der Ansprüche 1 oder 2, wobei das, eines, mehrere oder sämtliche der Alkylendiole ausgewählt sind aus der Gruppe bestehend aus Ethylenglykol, Propylenglykol und Hexylenglykol.

4. Mischung nach einem der vorangehenden Ansprüche, wobei das Gewichtsverhältnis von d,I-Menthol zu I-Menthol größer ist als 1 : 4, vorzugsweise größer als 1 : 2.

5. Mischung nach einem der vorangehenden Ansprüche, umfassend
- racemisches d,I-Menthol in einer Menge von 72,0 bis 85,0 Gew.-%,
- d,I-Isomenthol in einer Menge von 7,5 bis 20,0 Gew.-%,
- 1,2-Propylenglykol in einer Menge von 7,0 bis 11,0 Gew.-%,
- weitere Inhaltsstoffe in einer Gesamtmenge von 0 bis 2,0 Gew.-%,
wobei die Gewichtsprozentangaben bezogen sind auf das Gesamtgewicht der Mischung.

6. Mischung nach einem der Ansprüche 1 bis 4, umfassend
- racemisches d,I-Menthol in einer Menge von 48 bis 57 Gew.-%
- zusätzlich I-Menthol in einer Menge von 21,0 bis 32,0 Gew.-%,
- d,I-Isomenthol in einer Menge von 8,0 bis 14,0 Gew.-%,
- 1,2-Propylenglykol in einer Menge von 7,0 bis 11,0 Gew.-%,
- weitere Inhaltsstoffe in einer Gesamtmenge von 0 bis 2,0 Gew.-%,
wobei die Gewichtsprozentangaben bezogen sind auf das Gesamtgewicht der Mischung.

7. Produkt
(i) umfassend eine Mischung nach einem der vorangehenden Ansprüche und weitere Bestandteile oder
(ii) herstellbar durch Mischen oder in Kontakt bringen einer Mischung nach einem der vorangehenden Ansprüche mit weiteren Bestandteilen,
wobei die weiteren Bestandteile des Produkts kein Menthol und/oder Isomenthol sowie vorzugsweise kein Alkylendiol enthalten.

8. Produkt nach Anspruch 7, wobei das Produkt ausgewählt ist aus der Gruppe bestehend aus Aromastoff-Zubereitungen, kosmetischen Zubereitungen, der Ernährung und/oder dem Genuss dienenden, vorzugsweise verzehrfertigen Zubereitungen, insbesondere Getränken und kaubaren Nahrungsmitteln, insbesondere Kaugummis und Kaubonbons, Mundhygieneprodukten und Zahnpflegeprodukten, insbesondere Zahnpasta und zahnpflegende Kaugummis.

9. Verfahren zur Herstellung eines mentholhaltigen Produkts, vorzugweise eines Produkts nach einem der Ansprüche 7 oder 8, umfassend die folgenden Schritte:
- Bereitstellen oder Herstellen einer Mischung nach einem der Ansprüche 1 bis 6,
- Mischen oder in Kontakt bringen der Mischung mit weiteren Zutaten,
wobei die weiteren Zutaten kein Menthol und/oder Isomenthol sowie vorzugsweise kein Alkylendiol enthalten.

10. Verfahren nach Anspruch 9, wobei das Mischen oder in Kontakt bringen der Mischung mit den weiteren Zutaten bei einer Temperatur erfolgt, die höher ist als der Schmelzpunkt der Mischung, aber niedriger ist als 25 °C, vorzugsweise niedriger ist als 23 °C.

11. Verfahren zur Herstellung einer Mischung, die bei einem Druck von 101325 Pa und einer Temperatur von 20 °C und höher flüssig ist und racemisches d,I-Menthol sowie optional zusätzliches I-Menthol umfasst, in einer Gesamtmenge von 72,0 bis 85,0 Gew.-% bezogen auf das Gesamtgewicht der Mischung, vorzugweise zur Herstellung einer Mischung nach einem der Ansprüche 1 bis 6,
mit folgenden Schritten:
- Bereitstellen oder Herstellen von insgesamt 72,0 bis 85,0 Gewichtsteilen des racemischen d,I-Menthols sowie optional des zusätzlichen I-Menthols,
- Mischen des racemischen d,I-Menthols sowie gegebenenfalls des zusätzlichen I-Menthols mit:
- d,I-Isomenthol in einer Menge von 7,5 bis 20,0 Gewichtsteilen,
- einem ersten Alkylendiol sowie optional einem oder mehreren weiteren Alkylendiolen in einer Gesamtmenge von 7,0 bis 11,0 Gewichtsteilen,
- weiteren Inhaltsstoffen in einer Gesamtmenge von 0 bis 2,0 Gewichtsteilen,
wobei die Mengenverhältnisse so eingestellt werden, dass die resultierende Mischung bei einem Druck von 101325 Pa und einer Temperatur von 20 °C und höher flüssig ist.

12. Verfahren nach Anspruch 11, wobei das erste Alkylendiol bzw. eines, mehrere oder sämtliche der Alkylendiole ausgewählt sind aus der Gruppe bestehend aus Alkylenglykolen mit 2 bis 6 C-Atomen.

13. Verfahren nach einem der Ansprüche 11 oder 12, wobei das, eines, mehrere oder sämtliche der Alkylendiole ausgewählt sind aus der Gruppe bestehend aus Ethylenglykol, Propylenglykol und Hexylenglykol.

14. Verfahren nach einem der Ansprüche 11 bis 13, wobei das Gewichtsverhältnis von d,I-Menthol zu I-Menthol größer ist als 1 : 4, vorzugsweise größer als 1 : 2.

15. Verwendung von d,I-Isomenthol im Gemisch mit einem ersten Alkylendiol sowie optional einem oder mehreren weiteren Alkylendiolen als Mittel zur Erniedrigung des Schmelzpunktes von d,I-Menthol, optional in Gegenwart zusätzlichen I-Menthols, vorzugweise auf eine Temperatur von 20 °C oder niedriger bei einem Druck von 101325 Pa.
